(19) Europäisches Patentamt
European Patent Office
Office européen des brevets

(11) Veröffentlichungsnummer: **0 012 970**
**B1**

(12) **EUROPÄISCHE PATENTSCHRIFT**

(45) Veröffentlichungstag der Patentschrift: **15.07.81**

(51) Int. Cl.³: **C 07 C 127/24,** C 08 G 18/80

(21) Anmeldenummer: **79105220.2**

(22) Anmeldetag: **17.12.79**

# (54) Neue Polyisocyanate, ein Verfahren zu ihrer Herstellung und ihre Verwendung.

(30) Priorität: **30.12.78 DE 2856864**

(43) Veröffentlichungstag der Anmeldung: **09.07.80 Patentblatt 80/14**

(45) Bekanntmachung des Hinweises auf die Patenterteilung: **15.07.81 Patentblatt 81/28**

(84) Benannte Vertragsstaaten: **BE DE FR GB IT NL**

(56) Entgegenhaltungen: **DE-A-2 010 887**

(73) Patentinhaber: **BAYER AG, Zentralbereich Patente, Marken und Lizenzen, D-5090 Leverkusen 1, Bayerwerk (DE)**

(72) Erfinder: **Reichmann, Wolfgang, Dr., Vohwinkelallee 19, D-4000 Düsseldorf 1 (DE)**
Erfinder: **König, Klaus, Dr., Heymannstrasse 50, D-5090 Leverkusen 1 (DE)**
Erfinder: **Schönfelder, Manfred, Dr., Höhenstrasse 126, D-5090 Leverkusen 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 012 970 B1**

# Neue Polyisocyanate, ein Verfahren zu ihrer Herstellung und ihrer Verwendung

Die vorliegende Erfindung betrifft neue, Harnstoff- und/oder Polyuretgruppen aufweisende organische Polyisocyanate mit aliphatisch bzw. cycloaliphatisch gebundenen Isocyanatgruppen, ein Verfahren zu ihrer Herstellung, sowie ihre Verwendung, gegebenenfalls in mit Blockierungsmittel für Isocyanatgruppen blockierter Form, zur Herstellung von Polyurethankunststoffen.

Biuretgruppen enthaltende Polyisocyanate sind bekannt und finden als Rohstoffe für hochwertige, lichtechte Lackierungen praktische Verwendung. Sie können beispielsweise aus Diisocyanaten und Wasser (DE-AS 1 101 394), Schwefelwasserstoff (DE-AS 1 165 580), Ameisensäure (DE-AS 1 174 760), tertiären Alkoholen (DE-AS 1 543 178, DE-AS 1 931 055) oder Monoaminen (DE-OS 2 308 015) hergestellt werden.

Bei diesen Verfahren des Standes der Technik entstehen aus einem Teil der Isocyanatgruppen zunächst Aminogruppen, die mit überschüssigem Diisocyanat über die entsprechenden Harnstoffdiisocyanate zu Biuretpolyisocyanaten weiterreagieren. Die Umwandlung der Isocyanatgruppen in Aminogruppen wird stets durch die Entstehung von gasförmigen Nebenprodukten wie Kohlendioxid, Kohlenmonoxid, Kohlensulfoxid oder Olefine begleitet, deren Beseitigung zu Abgasproblemen führen kann. Bei der heterogenen Reaktion von Diisocyanaten mit Wasser besteht zusätzlich die Gefahr der Bildung von unlöslichen Polyharnstoffen, die nur schwer abzutrennen sind. Von besonderem Nachteil ist jedoch der Umstand, dass bei diesen Verfahren zunächst ein Teil der Isocyanatgruppen des als Ausgangsmaterial verwendeten Diisocyanats unter Aminbildung vernichtet wird.

Es mangelte daher auch nicht an Versuchen, Biuretgruppen aufweisende Polyisocyanate durch direkte Umsetzung von Diaminen mit Diisocyanaten ohne Abspaltung von flüchtigen Nebenprodukten und ohne Vernichtung von Isocyanatgruppen für Aminbildung herzustellen.

Wegen der hohen Reaktivität von aliphatischen Aminogruppen gegenüber Isocyanatgruppen traten insbesondere bei Umsetzungen von primären Diaminen mit Diisocyanaten erhebliche praktische Schwierigkeiten auf, da die Tendenz zur Bildung von unlöslichen Polyharnstoffen und vernetzten Produkten sehr gross ist.

Wie aus der DE-OS 2 261 065 beispielsweise hervorgeht, ist zur Vervollständigung der Umsetzung bei technisch leicht zugänglichen Ausgangsmaterialien wie Hexamethylendiamin und Hexamethylendiisocyanat ein unwirtschaftlich langes Nachheizen bei hoher Temperatur notwendig. Dies führt zu einer starken Beeinträchtigung der Eigenschaften der Verfahrensprodukte, insbesondere deren Eigenfarbe.

Diese Nachteile können nach dem Verfahren der DE-OS 2 609 995 beseitigt werden, wenn das Diamin unter besonderen Vorkehrungen dampfförmig in das Diisocyanat eingeleitet wird. Bei diesem Verfahren muss jedoch sehr darauf geachtet werden, dass kein Diisocyanat in das Einleitungsrohr gelangt, weil sonst schnell durch Harnstoffbildung verursachte Verstopfungen auftreten können.

Von gewissem Erfolg waren auch Verfahren, die sich spezieller Ausgangsmaterialien bedienten. So müssen gemäss der DE-PS 1 215 365 als Diamin-Komponente höhermolekulare Diaminopolyäther eingesetzt werden, um die Bildung von schwerlöslichen Nebenprodukten auszuschalten. Das Verfahren der DE-OS 1 963 190 ist seinerseits auf die Verwendung von diprimären aromatischen Diaminen mit durch sterische oder elektronische Effekte verminderter Reaktivität beschränkt. Nach der GB-PS 1 078 390 kann die Bildung von unlöslichen Polyharnstoffen bei der direkten Umsetzung von primären Diaminen mit Diisocyanaten unter Mitverwendung eines Lösungsmittels, wie z.B. Chloroform, verhindert werden. Diese Lehre bezieht sich auch nur auf aromatische Diamine und hat den Nachteil, dass im Anschluss an die Reaktion das Lösungsmittel wieder entfernt werden muss.

Bei diesen Verfahren des Standes der Technik finden während der Bildung der Biuretpolyisocyanate dauernde Umgruppierungsreaktionen statt, durch die in Abhängigkeit vom $NCO/NH_2$-Verhältnis das eingesetzte Diamin in das entsprechende Diisocyanat übergeht. Dadurch fallen bei der Abtrennung des nichtumgesetzten Diisocyanats vom Biuretpolyisocyanat als Destillate Gemische verschiedener Diisocyanate an, falls nicht Diamine und Diisocyanate gleicher Konstitution verwendet werden. Weiterhin nachteilig hierbei ist, dass im Biuretpolyisocyanat selbst mehr oder weniger grosse Mengen des sich durch Umbiuretisierung aus dem Diamin bildenden Diisocyanats als Monomerenanteil zurückbleiben.

Gemäss der DE-OS 2 010 887 ist die direkte Umsetzung von sekundären Diaminen insbesondere mit aromatischen Diisocyanaten durchführbar. Nach dem dort beschriebenen Verfahren hergestellte Produkte mit aromatisch gebundenen Isocyanatgruppen sind jedoch für hochwertige und lichtechte Lackierungen ungeeignet.

Es war daher die Aufgabe der vorliegenden Erfindung, ein neues Verfahren zur Verfügung zu stellen, welches auf einfache Weise die Herstellung von hochwertigen modifizierten aliphatischen Polyisocyanaten gestattet, die die Vorteile der bekannten Biuretpolyisocyanate in sich vereinigen, ohne dass das Verfahren mit den genannten Nachteilen der Verfahren des Standes der Technik behaftet ist.

Diese Aufgabe konnte überraschenderweise dadurch gelöst werden, dass man bestimmte, nachstehend näher beschriebene aliphatische Diamine mit überschüssigen Mengen an bestimmten, nachstehend näher beschriebenen Diisocyanaten unter bestimmten, nachstehend näher beschriebenen Reaktionsbedingungen umsetzt.

Gegenstand der vorliegenden Erfindung sind neue Polyisocyanate der Formel

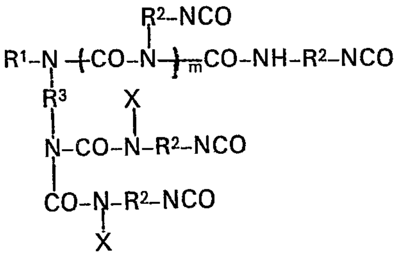

in welcher

$R^1$ für einen aliphatischen Kohlenwasserstoffrest mit 1 bis 20 Kohlenstoffatomen oder einen cycloaliphatischen Kohlenwasserstoffrest mit 4 bis 20 Kohlenstoffatomen steht,

die jeweiligen Reste $R^2$ und $R^3$ für gleiche oder verschiedene Reste stehen und gegebenenfalls Estergruppen enthaltende aliphatische Kohlenwasserstoffreste mit insgesamt 2 bis 20 Kohlenstoffatomen oder cycloaliphatische Kohlenwasserstoffreste mit 4 bis 20 Kohlenstoffatomen bedeuten, wobei jeweils zwischen den beiden Stickstoffatomen mindestens 2 Kohlenstoffatome angeordnet sind,

X für einen Rest der Formel

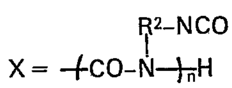

steht und

m und n jeweils für 0, oder eine Zahl zwischen 0 und 2 stehen.

Gegenstand der vorliegenden Erfindung ist auch ein Verfahren zur Herstellung dieser neuen Polyisocyanate durch Umsetzung von im Sinne der Isocyanat-Additionsreaktion aktive Wasserstoffatome aufweisenden organischen Diaminen mit überschüssigen Mengen an organischen Diisocyanaten der Formel

$R^2(NCO)_2$

dadurch gekennzeichnet, dass man als Diamine solche der Formel

$R^1\text{-}NH\text{-}R^3\text{-}NH_2$

verwendet, wobei die Reste $R^1$, $R^2$ und $R^3$ die obengenannte Bedeutung haben.

Gegenstand der vorliegenden Erfindung ist auch eine Abänderung dieses Verfahrens, welche dadurch gekennzeichnet ist, dass man organische Diamine der Formel

$R^1\text{-}NH\text{-}R^3\text{-}NH_2$

in einem ersten Reaktionsschritt mit überschüssigen Mengen eines ersten Diisocyanats der Formel

$R^2(NCO)_2$

zur Reaktion bringt, und anschliessend das so erhaltene, eine Harnstoff- und eine Biuretgruppe aufweisende Umsetzungsprodukt in einem zweiten Reaktionsschritt mit einem zweiten Diisocyanat der Formel

$R^2(NCO)_2$

umsetzt, wobei $R^1$, $R^2$ und $R^3$ die obengenannte Bedeutung haben, und wobei $R^2$ des ersten Diisocyanats von $R^2$ des zweiten Diisocyanats verschieden ist.

Gegenstand der vorliegenden Erfindung ist schliesslich auch die Verwendung der neuen Polyisocyanate, gegebenenfalls in mit Blockierungsmittel blockierter Form als Isocyanatkomponente bei der Herstellung von Polyurethankunststoffen nach dem Isocyanat-Polyadditionsverfahren.

In obigen Formeln und auch nachstehend haben $R^1$, $R^2$, $R^3$, X, m und n die bereits genannte Bedeutung. Die bevorzugte Bedeutung dieser Variablen ist die folgende:

$R^1$ steht vorzugsweise für einen aliphatischen Kohlenwasserstoffrest mit 1 bis 4 Kohlenstoffatomen,

$R^2$ bedeutet vorzugsweise einen aliphatischen Kohlenwasserstoffrest mit 6 bis 10 Kohlenstoffatomen oder einen cycloaliphatischen Kohlenwasserstoffrest mit 6 bis 15 Kohlenstoffatomen, wobei vorzugsweise alle Reste $R^2$ die gleiche Bedeutung haben.

$R^3$ steht vorzugsweise für einen aliphatischen Kohlenwasserstoffrest mit 2 bis 10 Kohlenstoffatomen oder einen cycloaliphatischen Kohlenwasserstoffrest mit 6 bis 10 Kohlenstoffatomen,

m und n stehen jeweils vorzugsweise für 0 oder 1.

Bei den erfindungsgemässen Polyisocyanaten der obengenannten Formel handelt es sich oft um Homologengemische, so dass m und n im statistischen Mittel oft auch gebrochene Zahlen im Bereich zwischen 0 und 2 darstellen können. Auch bei der bevorzugten Bedeutung der Reste $R^2$ und $R^3$ sind zwischen den beiden Stickstoffatomen jeweils mindestens 2 Kohlenstoffatome angeordnet.

Ausgangsmaterialien für das erfindungsgemässe Verfahren sind eine primäre und eine sekundäre Aminogruppe aufweisende, ansonsten jedoch unter den Bedingungen des erfindungsgemässen Verfahrens inerte aliphatische Diamine der allgemeinen Formel

$R_1\text{-}NH\text{-}R_3\text{-}NH_2.$

Vorzugsweise werden solche Diamine der genannten Formel eingesetzt, die durch Cyanäthylierung von primären Monoaminen und nachfolgender Hydrierung gewonnen werden. Konkrete Beispiele für derartige Diamine sind:

1-Amino-3-methylaminopropan, 1-Amino-3-ethylaminopropan, 1-Amino-3-propylaminopropan.

Es können jedoch auch auf andere Weise hergestellte Vertreter dieser Substanzklasse eingesetzt werden. Beispiele hierfür sind:
N-Methylethylendiamin, N-Ethylethylendiamin, 1-Amino-4-methylaminobutan, 1-Amino-6-methylaminohexan, oder 2-Amino-6-[(N-methyl)-amino]-hexancarbonsäure-(1)-ethylester. Beliebige Gemische der genannten Diamine können ebenfalls verwendet werden.

Besonders bevorzugt wird 1-Amino-3-methylaminopropan eingesetzt.

Weitere Ausgangsmaterialien für das erfindungsgemässe Verfahren sind Diisocyanate der Formel

$R^2(NCO)_2$.

Typische Beispiele derartiger Diisocyanate sind 1,4-Diisocyanatobutan, 1,6-Diisocyanatohexan, 2,4,4-Trimethyl-1,6-diisocyanatohexan, 1,11-Diisocyanatoundecan, 3-Isocyanatomethyl-3,5,5-trimethylcyclohexylisocyanat, 4,4'-Cyclohexandiisocyanat, 4,4'-Dicyclohexylmethandiisocyanat, 1,2-Bis-(isocyanatomethyl)-cyclobutan oder 6-Isocyanatocapronsäure-2-isocyanatoäthylester. Bevorzugt wird Hexamethylendiisocyanat eingesetzt.

Wie bereits oben ausgeführt entstehen bei der einfachen Umsetzung von einfachen organischen Diaminen wie Hexamethylendiamin und einfachen Diisocyanaten wie z.B. Hexamethylendiisocyanat sofort in dem stets angewandten Diisocyanatüberschuss schwerlösliche Polyharnstoffe, die nur durch langzeitiges Erhitzen in Gegenwart des Diisocyanat-Überschusses in entsprechende Biuretpolyisocyanate überführt werden können. Dieses langzeitige Erhitzen hat jedoch eine unerwünschte Verfärbung der Reaktionsprodukte und andere negative Begleiterscheinungen zur Folge. Es war nun sehr überraschend festzustellen, dass bei Verwendung der erfindungswesentlichen Diamine als Ausgangsmaterialien diese Schwierigkeiten weitgehend unterdrückt werden, so dass sofort, auch in Abwesenheit von Katalysatoren, erfindungsgemässe Polyisocyanate der obengenannten Formel entstehen, in welcher m und n in erster Näherung jeweils für 0 stehen. In Abwesenheit von Katalysatoren sind diese eine Harnstoff- und eine Biuretgruppe aufweisenden Triisocyanate gegenüber weiteren Mengen an Ausgangsdiisocyanat sehr reaktionsträge. Wie jedoch überraschend gefunden wurde gelingt es, diese Reaktionsträgheit durch Mitverwendung geeigneter Katalysatoren zu überwinden. Bei diesen erfindungsgemäss einzusetzenden Katalysatoren handelt es sich um protonenabspaltende starke Säuren, welche mit Isocyanaten, insbesondere mit aliphatischen oder cycloaliphatischen Isocyanaten, unter Bildung eines gemischten Säureanhydrids reagieren, wobei die dem Isocyanat entsprechende Carbamidsäure und die protonenabspaltende Säure die Säuren des gemischten Säureanhydrids darstellen. So reagieren derartige, für das erfindungsgemässe Verfahren geeignete Säuren HY (Y = Säurerest nach Abspaltung des Protons) mit Isocyanaten Z-NCO zu Addukten der Formel Z-NH-CO-Y, welche als gemischtes Anhydrid der Carbamidsäure Z-NH-COOH und der Säure HY anzusehen sind.

Beispiele geeigneter Säuren sind Halogenwasserstoffe wie z.B. Fluorwasserstoff, Chlorwasserstoff, Bromwasserstoff oder Jodwasserstoff, Chlorsulfonsäure, Fluorsulfonsäure, Schwefelsäure, Alkansulfonsäuren wie z.B. Methansulfonsäure oder perhalogenierte Alkansulfonsäuren wie z.B. Trifluormethansulfonsäure. Chlorwasserstoff ist die beim erfindungsgemässen Verfahren bevorzugt einzusetzende Säure. Anstelle der Säuren können beim erfindungsgemässen Verfahren selbstverständlich sowohl die den Säuren entsprechenden Ammoniumsalze mit den als Ausgangsmaterial eingesetzten Aminen oder die den Säuren entsprechenden gemischten Carbamidsäureanhydride, insbesondere Carbamidsäurechloride, der als Ausgangsmaterial eingesetzten Diisocyanate oder eines beliebigen anderen Isocyanats eingesetzt werden. Im allgemeinen werden die Katalysatoren in Mengen von 0,001 bis 10, vorzugsweise 0,01 bis 1,0 Gew.-%, bezogen auf das Gesamtgewicht der Reaktionspartner, eingesetzt.

Die Durchführung des erfindungsgemässen Verfahrens ist bei Temperaturen im Bereich von ca. 20 bis 250°C möglich. Ohne Verwendung der beispielhaft genannten Katalysatoren wird das Verfahren vorteilhafterweise bei 140 bis 180°C durchgeführt, wobei Harnstoff- und Biuretgruppen aufweisende Verfahrensprodukte (m, n = 0) entstehen. Unter Verwendung der beispielhaft genannten Säuren als Katalysatoren ist die über diese Zwischenstufe hinausführende Reaktion zu Polyuretgruppen aufweisenden Additionsprodukten bei Temperaturen im Bereich von 20 bis 160°C durchführbar, wobei die Umsetzung im allgemeinen im Bereich zwischen 90 und 140°C rasch abläuft. Die erfindungsgemässe Katalyse gestattet somit unter milden Reaktionsbedingungen die Herstellung von Polyuretgruppen aufweisenden Isocyanat-Additionsprodukten aus aliphatischen Diisocyanaten und aliphatischen, eine primäre und eine sekundäre Aminogruppe nebeneinander aufweisenden, Diaminen, bzw. aus, eine primäre und eine sekundäre Aminogruppe nebeneinander aufweisenden Diaminen und Diisocyanaten entstehenden, Harnstoffgruppen und Biuretgruppen aufweisenden Verbindungen. Es ist selbstverständlich, dass die Katalysatorzugabe auch zu beliebigen Zeitpunkten während der Umsetzung erfolgen kann. So kann beispielsweise erst, ohne Katalysatorverwendung, zur Förderung der Löslichkeit von Harnstoffgruppen aufweisenden Zwischenstufen bei höherer Temperatur gearbeitet werden, wobei schnell klare, Harnstoff- und Biuretgruppen aufweisende Verfahrensprodukte (m, n = 0) enthaltende Reaktionslösungen entstehen, die dann bei niedrigerer Temperatur, jetzt aber unter Katalysatormitwirkung, in Polyuretgruppen aufweisende Verfahrensprodukte übergeführt werden. Bei

der Durchführung des erfindungsgemässen Verfahrens unter Verwendung von eine primäre und eine sekundäre Aminogruppe aufweisenden Diaminen als Ausgangsmaterialien entstehen keine leicht flüchtigen Nebenprodukte. Der bezüglich der erfindungsgemässen Umsetzung indifferente Rest der Diamine bildet stets einen nicht flüchtigen Bestandteil der erfindungsgemässen Verfahrensprodukte. Die durch Umbiuretisierung mögliche Umwandlung der primären Aminogruppe der beispielhaft genannten Diamine in eine Isocyanatgruppe kann zusätzlich zu einer Erhöhung des Isocyanatgehalts der Verfahrensprodukte beitragen.

Bei der Durchführung des erfindungsgemässen Verfahrens unter Verwendung von eine primäre und eine sekundäre Aminogruppe aufweisenden Diaminen als Ausgangsmaterialien werden diese und die Diisocyanate im allgemeinen in Mengenverhältnissen eingesetzt, die einem NCO/NH-Molverhältnis von 2:1 bis 100:1, vorzugsweise 2,5:1 bis 30:1, entsprechen. Bei Verwendung von Harnstoffgruppen und Biuretgruppen aufweisenden Ausgangsmaterialien gehen die in diesen gebundenen erfindungsgemäss verwendeten Diamine mit in die Berechnung des NCO/NH-Molverhältnisses ein.

Zur Durchführung des erfindungsgemässen Verfahrens wird im allgemeinen wie folgt verfahren:

Man legt das Diisocyanat in einem geeigneten Reaktionsgefäss vor und gibt das Diamin bei Temperaturen von 140 bis 180 °C zu. Dabei beträgt das NCO/NH-Molverhältnis im allgemeinen 2,5:1 bis 30:1. Während der Aminzugabe tritt eine schwache Trübung der Reaktionslösung auf, die nach erfolgter Aminzugabe rasch verschwindet. Es entsteht eine klare Harnstoff- und Biuretgruppen aufweisende Polyisocyanate (m, n = 0) enthaltende Lösung.

Soll die Reaktion nicht an dieser Stelle beendet werden, so wird die Reaktionslösung auf eine Temperatur von 90 bis 140 °C gebracht und mit einem der erfindungsgemäss genannten Katalysatoren versetzt, um die Bildung von höheren Polyuretgruppen in Gang zu setzen. Bei der Herstellung von auch höhere Polyuretgruppen aufweisenden Polyisocyanaten kann die Katalysatorzugabe natürlich zu jedem Zeitpunkt der Reaktion erfolgen. Man kann ihn z.B. mit dem Diisocyanat vorlegen, ihn als Ammoniumsalz mit den Aminen eindosieren oder ihn erst nach Beendigung der Vorreaktion zu Harnstoff- oder Biuretgruppen aufweisenden Zwischenstufen zugeben. Danach wird das Reaktionsgemisch bei einer Temperatur im Bereich von 90 bis 140 °C gehalten und der Reaktionsverlauf durch Kontrolle der Isocyanatgehaltsabnahme verfolgt. Bei Verwendung von flüchtigen Katalysatoren, z.B. Chlorwasserstoff, kann zur Vermeidung von bei höheren Temperaturen möglichen Katalysatorverlusten unter Druck gearbeitet werden.

Wenn die NCO-Abnahme dem gewünschten «Polyuretisierungsgrad» entspricht: d.h., wenn pro Aminogruppe die gewünschte Menge an NCO-Gruppen umgesetzt ist, wird die Reaktion beendet. Dies geschieht einfach durch Abkühlen des Reaktionsgemisches auf 20 bis 50 °C. Die benötigten Reaktionszeiten hängen von der Art der Ausgangsprodukte, von der Temperatur und insbesondere von der Art und Menge des Katalysators ab. Sie betragen im allgemeinen 1 bis 5, vorzugsweise 1 bis 2 Stunden. Nach Beendigung der Reaktion erhält man klare, farblose bis schwach gelblich gefärbte Reaktionslösungen.

Die Reaktionen werden meistens zu einem Zeitpunkt beendet, zu dem im Mittel, bezogen auf die primäre und sekundäre Aminogruppe, der erfindungsgemäss verwendeten Diamine insgesamt ca. 4 NCO-Gruppen verbraucht sind. Es ist jedoch möglich, einen höheren «Polyuretierungsgrad» zu erreichen, d.h. bezogen auf die Summe der Aminogruppen 5 und mehr NCO-Gruppen umzusetzen. Allerdings nehmen die Viskositäten der Produkte dann schnell zu.

Der Katalysator wird im allgemeinen durch Andestillieren des Reaktionsgemisches im Vakuum entfernt. Bei Verwendung von Halogenwasserstoffen als Katalysatoren kann die Beseitigung, insbesondere bei kleineren Katalysatormengen auch durch Zugabe äquimolarer Mengen Propylenoxid erfolgen. Ferner ist es möglich, den Katalysator durch z.B. Dünnschichtverdampfung zu entfernen, falls das Rohisocyanat von überschüssigem Diisocyanat befreit wird. Das Destillat der Dünnschichtdestillation, das dann neben dem Diisocyanat den Katalysator enthält, kann als Ausgangsmaterial wiederverwendet werden.

Ist die Entfernung von überschüssigem Diisocyanat vorgesehen, so erfolgt sie meistens durch Dünnschichtverdampfung; sie kann jedoch auch durch Extraktion mit geeigneten Lösungsmitteln, wie z.B. Hexan, Heptan etc. erreicht werden.

Die Rohisocyanate können als solche verwendet werden. In den meisten Fällen werden sie jedoch vorzugsweise durch Dünnschichtverdampfung oder Extraktion von monomeren Isocyanatanteilen befreit. Die monomerenfreien Produkte sind hellgelbe Öle oder auch feste Harze; der NCO-Gehalt beträgt 5 bis 22 Gew.-%.

Das Verfahren eignet sich vorzüglich für die kontinuierliche Durchführung. In diesen Fällen werden z.B. mehrere Reaktionsgefässe in Form einer Kaskade hintereinander geschaltet. Im ersten Reaktionsgefäss, das auch durch einen temperaturregulierten statischen Mischer ersetzt werden kann, werden die Ausgangsprodukte Diisocyanat und Diamin bei 140 bis 180 °C vermischt. Im zweiten Reaktionsgefäss wird bei 90 bis 140 °C der Katalysator zugegeben, falls Reaktionsprodukte mit höherem Polyuretisierungsgrad erwünscht sind. In diesem Fall findet im dritten und gegebenenfalls in weiteren Reaktionsgefässen die Weiterreaktion zum Polyuretpolyisocyanat statt, wobei durch Steuerung der Temperatur und der Verweilzeit der angestrebte «Polyuretisierungsgrad» eingestellt wird. Überschüssiges Diisocyanat und der Katalysator werden z.B. über einen Schlangenrohrverdampfer kombiniert mit nachgeschaltetem Dünnschichtverdampfer entfernt. Die aus Diisocyanat und Katalysator bestehenden Destillate werden vereinigt, gewünschtenfalls vom Katalysator befreit und wieder in den Prozess zurückgeführt. Das Polyisocyanat wird als Rückstand der Dünnschichtdestillation gewonnen.

Bei der Durchführung des erfindungsgemässen Verfahrens können die Eigenschaften der erhaltenen modifizierten Polyisocyanate, insbesondere deren NCO-Funktionalität, NCO-Gehalt, sowie die Viskosität nicht nur durch Wahl der geeigneten Ausgangsmaterialien, sondern besonders einfach durch Einstellung des «Polyuretisierungsgrades», d.h. der Zahl der pro Aminogruppe umgesetzten NCO-Gruppen gesteuert werden.

Gemäss einer besonderen Ausführungsform des erfindungsgemässen Verfahrens werden in der ersten, in Abwesenheit von Katalysatoren, durchgeführten Reaktionsstufe Diisocyanate der Formel

$R^2(NCO)_2$

eingesetzt, das erhaltene eine Harnstoff- und eine Biuretgruppe aufweisende Polyisocyanat vom überschüssigen Diisocyanat befreit und anschliessend einer katalytischen Weiterreaktion unter Verwendung eines anderen Diisocyanats der Formel

$R^2(NCO)_2$

unterzogen. Hierbei haben selbstverständlich die Reste $R^2$ eine verschiedenartige Bedeutung. Auf diese Weise ist es möglich, erfindungsgemässe Polyisocyanate mit unterschiedlichen Resten $R^2$ herzustellen.

Die erfindungsgemässen Verfahrensprodukte können insbesondere als Isocyanatkomponente bei der Herstellung von Polyurethankunststoffen nach dem Isocyanat-Polyadditionsverfahren eingesetzt werden. Sie eignen sich sowohl zur Herstellung von Polyurethanschaumstoffen, als auch zur Herstellung von Elastomeren, Beschichtungen oder Verklebungen. Insbesondere bei Verwendung der erfindungsgemässen Verfahrensprodukte für das erstgenannte Einsatzgebiet erübrigt sich oft ein Abdestillieren des überschüssigen Diisocyanats nach Beendigung der erfindungsgemässen Umsetzung. Die monomerenfreien erfindungsgemässen Verfahrensprodukte stellen hervorragende Rohstoffe zur Herstellung hochwertiger, wetterfester und lichtechter Lackierungen dar. Bei der Verwendung der erfindungsgemässen Verfahrensprodukte als Lackpolyisocyanate kommen sie oft in mit an sich bekannten Blockierungsmitteln blockierter Form zum Einsatz. Derartige Blockierungsmittel sind beispielsweise Malonsäurediethylester, ε-Caprolactam oder Acetessigesterethylester.

Die nachfolgenden Beispiele dienen der näheren Erläuterung der Erfindung. Alle Prozentangaben beziehen sich, soweit nicht anderslautend vermerkt, auf Gewichtsprozente.

Beispiel 1

In einem 4 l-Vierhalskolben mit Rührer, Rückflusskühler und Kontaktthermometer wurden zu 3696 g (22 Mol) 1,6-Diisocyanatohexan 88 g (1 Mol) 1-Amino-3-methylaminopropan bei 140 bis 160 °C innerhalb von 1 Stunde zugetropft. Dabei wurde mit Hilfe einer besonderen Stickstoffüberlagerung dafür gesorgt, dass das eintropfende Amin nicht mit Isocyanatdämpfen in Berührung kam, bevor es in die Reaktionslösung gelangte. Während des Zutropfens trat eine schwache Trübung des Reaktionsgemisches auf, die schnell in Lösung ging. Nun wurde die Reaktionstemperatur auf 110 bis 120 °C gebracht und die Mischung mit 4 g Chlorwasserstoff versetzt. Nach einer Stunde betrug der NCO-Gehalt der Mischung 44,3% (entsprechend einem Verbrauch von 4,1 NCO-Gruppen, bezogen auf die Gesamtzahl der Aminogruppen, d.h. m und n entsprechen im statistischen Mittel jeweils (4,1-3) : 3 = 0,37). Die Reaktionslösung wurde auf Raumtemperatur abgekühlt. Die nachfolgende Dünnschichtdestillation des klaren Reaktionsgemisches ergab 700 g eines Polyisocyanats mit einem NCO-Gehalt von 20,0% und einer Viskosität von 11 500 mPa·s bei 20 °C.

Beispiel 2

Analog Beispiel 1 wurden 2856 g (17 Mol) 1,6-Diisocyanatohexan mit 88 g (1 Mol) 1-Amino-3-methylaminopropan bei 160 bis 170 °C innerhalb von 30 Minuten umgesetzt. Nach der Aminzugabe wurde ein klares Reaktionsgemisch erhalten. Die Weiterreaktion zum Polyuretpolyisocyanat wurde bei 100 bis 110 °C unter Zusatz von 1,5 g Chlorwasserstoff durchgeführt. Nach 1 Stunde war der NCO-Gehalt der Mischung auf 42,8% gesunken (entsprechend einem Verbrauch von 4 NCO-Gruppen, bezogen auf die Gesamtzahl der Aminogruppen). Nach der Dünnschichtdestillation wurden 700 g Polyisocyanat mit einem NCO-Gehalt von 19,6% und einer Viskosität von 17 500 mPa·s bei 20 °C erhalten.

Beispiel 3

Analog Beispiel 1 wurden 2016 g (12 Mol) 1,6-Diisocyanatohexan mit 88 g (1 Mol) 1-Amino-3-methylaminopropan bei 170 bis 180 °C innerhalb 1 Stunde umgesetzt. Nach dem Abkühlen auf 100 bis 110 °C wurde die klare Reaktionsmischung mit 1 g Chlorwasserstoff versetzt. Nach 1 Stunde betrug der NCO-Gehalt 39,9% (entsprechend einem Verbrauch von 4 NCO-Gruppen, bezogen auf die Gesamtzahl der Aminogruppen). Die Dünnschichtdestillation lieferte 630 g Polyisocyanat (NCO-Gehalt = 19,5%; Viskosität bei 20 °C = 25 400 mPa·s).

Beispiel 4

Analog Beispiel 1 wurden zu 3696 g (22 Mol) 1,6-Diisocyanatohexan, die 2 g Chlorwasserstoff enthielten, 88 g (1 Mol) 1-Amino-3-methylaminopropan bei 120 bis 140 °C innerhalb von 2 Stunden zugetropft. Während des Zutropfens bildeten sich in geringem Ausmass Festkörper, der bei der

Nachreaktion wieder in Lösung ging. Nach 2 Stunden war die Reaktionsmischung vollkommen klar. Der NCO-Gehalt war 44,4% (entsprechend einem Verbrauch von 4 NCO-Gruppen, bezogen auf die Gesamtzahl der Aminogruppen). Bei diesem Beispiel wurde auf die weitere Aufarbeitung verzichtet.

Beispiel 5

Analog Beispiel 1 wurden 3696 g (22 Mol) 1,6-Diisocyanatohexan mit 88 g (1 Mol) 1-Amino-3-methylaminopropan bei 150 bis 160 °C innerhalb von 1 Stunde umgesetzt. Nach Aufklaren der Reaktionslösung wurde die Temperatur auf 100 bis 120 °C gesenkt. Nun erfolgte die Zugabe von 2 g Methansulfonsäure. Nach 3 Stunden betrug der NCO-Gehalt 44,5% (entsprechend einem Verbrauch von 3,9 NCO-Gruppen, bezogen auf die Gesamtzahl der Aminogruppen). Der Ansatz wurde nicht weiter aufgearbeitet.

Beispiel 6

Analog Beispiel 1 wurden 2856 g (17 Mol) 1,6-Diisocyanatohexan mit 88 g (1 Mol) 1-Amino-3-methylaminopropan bei 160 bis 170 °C innerhalb von 30 Minuten umgesetzt. Der NCO-Gehalt der klaren Reaktionslösung betrug 44,0% (entsprechend einem Verbrauch von 3,1 NCO-Gruppen, bezogen auf die Gesamtzahl der Aminogruppen. Der Ansatz wurde sofort auf Raumtemperatur abgekühlt. Durch anschliessende Dünnschichtdestillation wurden 520 g eines Biuretpolyisocyanats mit einem NCO-Gehalt von 19,9% und einer Viskosität von 7000 mPa·s bei 20 °C gewonnen.

Beispiel 7

Analog Beispiel 1 wurden in einem 6 l-Kolben 4884 g (22 Mol) Isophorondiisocyanat mit 88 g (1 Mol) 1-Amino-3-methylaminopropan bei 150 bis 160 °C innerhalb 1 Stunde umgesetzt. Der NCO-Gehalt der klaren Reaktionslösung betrug 34,6% (entsprechend einem Verbrauch von 3,1 NCO-Gruppen, bezogen auf die Gesamtzahl der Aminogruppen). Auf die Entfernung von überschüssigem Diisocyanat wurde verzichtet.

Beispiel 8 (Verwendungsbeispiel)

77 g einer 65%igen Lösung eines stark verzweigten Polyesters auf Basis Phthalsäureanhydrid und Trimethylolpropan (Hydroxylgehalt 8%) in Äthylglycolacetat/Xylol (1:1) wurden nach Zugabe von 0,5 g eines tertiären Amins als Katalysator und 0,4 g Cellulosebutyrat-propionat als Verlaufmittel mit 100 g eines Lösungsmittelgemisches aus Methyläthylketon, Butylacetat, Äthylglycolacetat und Toluol (4:1:4:1) verdünnt. Hierzu wurden 65,9 g einer 75%igen Lösung des Polyisocyanats aus Beispiel 1 in Äthylglycolacetat/Xylol (1:1) gegeben (NCO/OH-Molverhältnis = 1:1). Die fertige Lacklösung wurde dann auf Stahlbleche aufgetragen, wo die Lackfilme bei Raumtemperatur aushärteten. Die durchgehärteten Klarlackfilme waren kratzfest, elastisch und gegen Lösungsmittel wie Toluol, Äthylglycolacetat, Äthylacetat oder Aceton beständig. Sie hatten ferner folgende Eigenschaften:

| | |
|---|---|
| Schichtdicke | ca. 40 μ |
| Erichsentiefung (DIN 53 156) | |
| nach 3 Tagen | 9,8 mm |
| nach 7 Tagen | 9,0 mm |
| Pendelhärte (DIN 53 157) | |
| nach 3 Tagen | 147 Sekunden |
| nach 7 Tagen | 169 Sekunden |

Beispiel 9 (Verwendungsbeispiel)

77 g einer 65%igen Lösung eines stark verzweigten Polyesters auf Basis Phthalsäureanhydrid und Trimethylolpropan (Hydroxylgehalt 8%) in Äthylglycolacetat/Xylol (1:1) wurden nach Zugabe von 0,5 g eines tertiären Amins als Katalysator und 0,4 g Cellulosebutyratpropionat als Verlaufmittel mit 100 g eines Lösungsmittelgemisches aus Methyläthylketon, Butylacetat, Äthylglycolacetat und Toluol (4:1:4:1) verdünnt. Hierzu wurden 65,9 g einer 75%igen Lösung des Polyisocyanats aus Beispiel 6 in Äthylglycolacetat/Xylol (1:1) gegeben (NCO/OH-Molverhältnis = 1:1). Die fertige Lacklösung wurde dann auf Stahlbleche aufgetragen, wo die Lackfilme bei Raumtemperatur aushärteten. Die durchgehärteten Klarlackfilme waren kratzfest, elastisch und gegen Lösungsmittel wie Toluol, Äthylglycolacetat, Äthylacetat oder Aceton beständig. Sie hatten ferner folgende Eigenschaften:

| | |
|---|---|
| Schichtdicke | ca. 50 μ |
| Erichsentiefung (DIN 53 156) | |
| nach 3 Tagen | 9,6 mm |
| nach 7 Tagen | 9,0 mm |
| Pendelhärte (DIN 53 157) | |
| nach 3 Tagen | 146 Sekunden |
| nach 7 Tagen | 182 Sekunden |

Beispiel 10 (Vergleichsbeispiel)

Es wurden 77 g der im Beispiel 8 beschriebenen Polyesterlösung mit 50 g Titandioxid (Rutiltyp) zu einer Paste verarbeitet. Dieser Paste wurden neben Katalysator und Verlaufmittel 50 g des schon beschriebenen Lösungsmittelgemisches zugefügt. Die so erhaltene Mischung wurde mit 65,9 g einer 75%igen Lösung des Polyisocyanats aus Beispiel 1 in Äthylglycolacetat/Xylol (1:1) versetzt und in dünner Schicht auf Stahlbleche aufgetragen. Die pigmenthaltigen Lackfilme härteten bei Raumtemperatur durch. Sie zeichneten sich durch Kratzfestigkeit und Lösungsmittelresistenz aus und hatten verglichen mit den Klarlackfilmen folgende Eigenschaften:

| | |
|---|---|
| Schichtdicke | ca. 50 μ |
| Erichsentiefung (DIN 53 156) | |
| nach 3 Tagen | 10,0 mm |
| Pendelhärte (DIN 53 157) | |
| nach 3 Tagen | 91 Sekunden |
| nach 7 Tagen | 120 Sekunden |

Beispiel 11 (Verwendungsbeispiel)

Es wurden 77 g der im Beispiel 8 beschriebenen

Polyesterlösung mit 50 g Titandioxid (Rutiltyp) zu einer Paste verarbeitet. Dieser Paste wurden neben Katalysator und Verlaufmittel 50 g des schon beschriebenen Lösungsmittelgemisches zugefügt. Die so erhaltene Mischung wurde mit 65,9 g einer 75%igen Lösung des Polyisocyanats aus Beispiel 6 in Äthylglycolacetat/Xylol (1:1) versetzt und in dünner Schicht auf Stahlbleche aufgetragen. Die pigmenthaltigen Lackfilme härteten bei Raumtemperatur durch. Sie zeichneten sich durch Kratzfestigkeit und Lösungsmittelresistenz aus und hatten verglichen mit den Klarlackfilmen folgende Eigenschaften:

| | |
|---|---|
| Schichtdicke | ca. 50 μ |
| Erichsentiefung (DIN 53 156) | |
| nach 3 Tagen | 10,1 mm |
| Pendelhärte (DIN 53 157) | |
| nach 3 Tagen | 90 Sekunden |
| nach 7 Tagen | 112 Sekunden |

**Patentansprüche**

1. Polyisocyanate der Formel

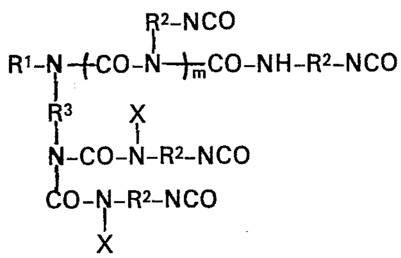

in welcher
$R^1$ für einen aliphatischen Kohlenwasserstoffrest mit 1 bis 20 Kohlenstoffatomen oder einen cycloaliphatischen Kohlenwasserstoffrest mit 4 bis 20 Kohlenstoffatomen steht,
die jeweiligen Reste $R^2$ und $R^3$ für gleiche oder verschiedene Reste stehen und gegebenenfalls Estergruppen enthaltende aliphatische Kohlenwasserstoffreste mit jeweils insgesamt 2 bis 20 Kohlenstoffatomen oder cycloaliphatische Kohlenwasserstoffreste mit 4 bis 20 Kohlenstoffatomen bedeuten, wobei jeweils zwischen den beiden Stickstoffatomen mindestens 2 Kohlenstoffatome angeordnet sind,
X für einen Rest der Formel

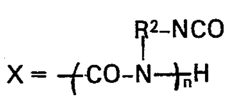

steht und
m und n jeweils für 0 oder eine Zahl zwischen 0 und 2 stehen.

2. Verfahren zur Herstellung von Polyisocyanaten gemäss Anspruch 1 durch Umsetzung von im Sinne der Isocyanat-Additionsreaktion aktive Wasserstoffatome aufweisenden organischen Diaminen mit überschüssigen Mengen an organischen Diisocyanaten der Formel

$R^2(NCO)_2$

dadurch gekennzeichnet, dass man als Diamine solche der Formel

$R^1\text{-}NH\text{-}R^3\text{-}NH_2$

verwendet, wobei
$R^1$, $R^2$ und $R^3$ die in Anspruch 1 genannte Bedeutung haben, wobei man zur Herstellung von Polyuretgruppen aufweisenden Additionsprodukten die Umsetzung in Gegenwart von katalytischen Mengen an starken, mit Isocyanaten gemischte Carbamidsäureanhydride bildenden Säuren durchführt.

3. Verfahren zur Herstellung von Polyisocyanaten gemäss Anspruch 1 durch Umsetzung von organischen Diaminen mit im Sinne der Isocyanat-Additionsreaktion aktiven Wasserstoffatomen mit überschüssigen Mengen an organischen Diisocyanaten, dadurch gekennzeichnet, dass man organische Diamine der Formel

$R^1\text{-}NH\text{-}R^3\text{-}NH_2$

in einem ersten Reaktionsschritt mit überschüssigen Mengen eines ersten Diisocyanats der Formel

$R^2(NCO)_2$

zur Reaktion bringt, und anschliessend das so erhaltene, eine Harnstoff- und eine Biuretgruppe aufweisende Umsetzungsprodukt in einem zweiten Reaktionsschritt mit einem zweiten Diisocyanat der Formel

$R^2(NCO)_2$

umsetzt, wobei
$R^1$, $R^2$ und $R^3$ die in Anspruch 1 genannte Bedeutung haben, mit der Massgabe, dass die Reste $R^2$ der beiden Diisocyanate voneinander verschieden sind,
wobei man die erste Reaktionsstufe in Abwesenheit von Katalysatoren und die zweite Reaktionsstufe in Gegenwart von katalytischen Mengen an starken, mit Isocyanaten gemischte Carbamidsäureanhydride bildenden Säuren durchführt.

4. Verwendung der Polyisocyanate gemäss Anspruch 1, gegebenenfalls in mit Blockierungsmittel für Polyisocyanate blockierter Form, als Isocyanatkomponente bei der Herstellung von Polyurethankunststoffen nach dem Isocyanat-Polyadditionsverfahren.

**Claims**

1. Polyisocyanates of the formula

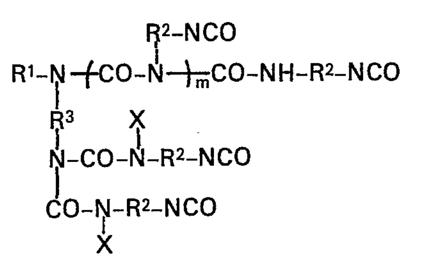

in which
$R^1$ represents an aliphatic hydrocarbon radical containing from 1 to 20 carbon atoms or a cycloaliphatic hydrocarbon radical containing from 4 to 20 carbon atoms, the respective radicals $R^2$ and $R^3$ represent equal or different radicals and denote aliphatic hydrocarbon radicals having in each case a total of 2 to 20 carbon atoms optionally containing ester groups or cycloaliphatic hydrocarbon radicals containing from 4 to 20 carbon atoms, at least two carbon atoms in each case being arranged between the two nitrogen atoms,
X represents a radical of the formula

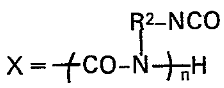

and
m and n are each 0 or a number from 0 to 2.

2. A process for the preparation of polyisocyanates according to claim 1 by reacting organic diamines containing hydrogen atoms which are active in the context of the isocyanate addition reaction with excess quantities of organic diisocyanates of the formula

$R^2(NCO)_2$

characterised in that the diamines used are those of the formula:

$R^1-NH-R^3-NH_2$

wherein $R^1$, $R^2$ and $R^3$ have the meaning given in claim 1, and for the preparation of addition products containing polyuret groups the reaction is carried out in the presence of catalytic quantities of strong acids forming mixed carbamic acid anhydrides with isocyanates.

3. A process for the preparation of the polyisocyanates according to claim 1 by reacting organic diamines containing hydrogen atoms which are active in the context of the isocyanate addition reaction with excess quantities of organic diisocyanates, characterised in that in a first reaction stage organic diamines of the formula

$R^1-NH-R^3-NH_2$

are reacted with excess quantities of a first diisocyanate of the formula

$R^2(NCO)_2$

and then the reaction product thus obtained which contains a urea group and a biuret group is reacted in a second reaction stage with a second diisocyanate of the formula

$R^2(NCO)_2$

wherein $R^1$, $R^2$ and $R^3$ have the meaning given in claim 1, with the proviso that the radicals $R^2$ of the two diisocyanates are different from one another, the first reaction stage being carried out in the absence of catalysts and the second reaction stage being carried out in the presence of catalytic quantities of strong acids forming mixed carbamic acid anhydrides with isocyanates.

4. The use of the polyisocyanates according to claim 1, optionally blocked with blocking agents for polyisocyanates, as isocyanate component in the production of polyurethane plastics by the isocyanate polyaddition process.

**Revendications**

1. Des polyisocyanates de formule:

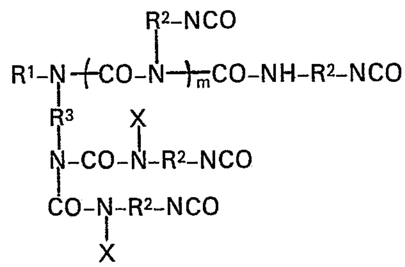

dans laquelle:
$R^1$ est un reste d'hydrocarbure aliphatique ayant 1 à 20 atomes de carbone ou un reste d'hydrocarbure cycloaliphatique ayant 4 à 20 atomes de carbone, les restes $R^2$ respectifs et $R^3$ sont des restes égaux ou différents et désignent des restes d'hydrocarbures aliphatiques portant éventuellement des groupes ester et ayant chacun au total 2 à 20 atomes de carbone ou des restes d'hydrocarbures cycloaliphatiques ayant 4 à 20 atomes de carbone, au moins deux atomes de carbone étant disposés dans chaque cas entre les deux atomes d'azote,
X est un reste de formule:

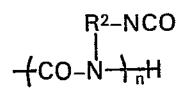

et
m et n sont égaux chacun à 0 ou à un nombre allant de 0 à 2.

2. Procédé de production de polyisocyanates suivant la revendication 1, par réaction de diamines organiques présentant des atomes d'hydrogène actifs dans le sens de la réaction d'addition d'un isocyanate avec des quantités en excès de diisocyanates organiques de formule:

$R^2(NCO)_2$

caractérisé en ce qu'on utilise comme diamines des composés de formule:

$R^1-NH-R^3-NH_2$

dans laquelle:
$R^1$, $R^2$ et $R^3$ ont la définition indiquée dans la revendication 1, en conduisant la réaction en présence de quantités catalytiques d'acides forts,

formant avec des isocyanates des anhydrides mixtes d'acide carbamique pour l'obtention de produits d'addition porteurs de groupes polyuret.

3. Procédé de production de polyisocyanates suivant la revendication 1, par réaction de diamines organiques portant des atomes d'hydrogène actifs dans le sens de la réaction d'addition d'un isocyanate avec des quantités en excès de diisocyanates organiques, caractérisé en ce qu'on fait réagir des diamines organiques de formule:

$R^1$-NH-$R^3$-$NH_2$

dans une première phase réactionnelle avec des quantités en excès d'un premier diisocyanate de formule:

$R^2(NCO)_2$

puis on fait réagir le produit réactionnel ainsi obtenu, portant un groupe urée et un groupe biuret, dans une seconde étape réactionnelle avec un second diisocyanate de formule:

$R^2(NCO)_2$

$R^1$, $R^2$ et $R^3$ ayant la définition donnée dans la revendication 1, à condition que les restes $R^2$ des deux diisocyanates soient différents l'un de l'autre, en conduisant la première étape réactionnelle en l'absence de catalyseurs et la seconde étape réactionnelle en présence de quantités catalytiques d'acides forts formant avec des isocyanates des anhydrides mixtes d'acide carbamique.

4. Utilisation des polyisocyanates suivant la revendication 1, éventuellement sous la forme protégée avec des agents de blocage pour des polyisocyanates, comme composant isocyanate dans la production de matières plastiques du type polyuréthanne par le procédé de polyaddition d'un isocyanate.